# FASCICULE DE BREVET EUROPEEN

(11) **EP 1 525 005 B1**
(45) Date de publication et mention de la délivrance du brevet: **10.09.2008**
(21) Numéro de dépôt: 03755600.8
(22) Date de dépôt: 15.07.2003
(51) Int. Cl.: A61L 11/00, B02C 19/00, B09B 3/00

(54) **DISPOSITIF ET METHODE DE PARCELLISATION ET DE DESINFECTION OU DE TRAITEMENT DE DECHETS**
VORRICHTUNG UND VERFAHREN ZUR FRAGMENTIERUNG UND DESINFEKTION ODER BEHANDLUNG VON ABFÄLLEN
DEVICE AND METHOD FOR WASTE BREAKDOWN AND DISINFECTION OR TREATMENT

(30) Priorité: 17.07.2002 FR 0209008
(43) Date de publication de la demande: 27.04.2005
(73) Titulaire: T.E.M. Technologies, Environnement et Médical, 31190 Auterive (FR)
(72) Inventeur: HENGL, Emmanuel, F-31450 ISSUS (FR); HENGL, Patrick, F-31450 ISSUS (FR)
(74) Mandataire: Jeannet, Olivier
(86) Numéro de dépôt international: PCT/FR2003/002223
(87) Numéro de publication internationale: WO 2004/009144

(56) Documents cités:
- EP-A- 0 277 507
- EP-A- 0 456 237
- EP-A- 0 597 779
- EP-A- 0 763 390
- WO-A-00/38744
- DE-U- 9 209 762
- FR-A- 2 793 143
- US-A- 4 578 185
- US-A- 5 213 774
- US-A- 5 364 589
- US-A- 5 570 845

## Description

La présente invention relève du domaine du traitement des déchets par broyage. Elle a plus particulièrement pour objet un dispositif et une méthode de parcellisation et de désinfection ou de traitement de déchets médicaux infectieux mais est également applicable au traitement de déchets putrescibles tels que des déchets alimentaires.

On connaît la technique de traitement de déchets médicaux consistant à broyer ces déchets pour en réduire le volume et à décontaminer ces déchets afin d'en éliminer les agents nocifs et infectieux.

Par exemple, le brevet FR 2 793 143 (HENGL) décrit un dispositif de broyage et de désinfection des déchets médicaux, qui comprend, en succession étagée, un compartiment d'alimentation, un compartiment de broyage et un compartiment de désinfection et d'évacuation des résidus.

Le compartiment d'alimentation comprend une trémie, intérieurement habillée d'une gaine déroulante prévue pour recevoir les déchets, qui sont acheminés vers le compartiment broyage au fur et à mesure du dévidement de la gaine au moyen de rouleaux d'entraînement.

Un premier inconvénient de ce dispositif réside dans l'ouverture de la capacité de réception des déchets formée par la gaine sur l'extérieur. Cet inconvénient est d'autant plus préjudiciable que le compartiment d'alimentation est en communication avec le compartiment de broyage, et, en conséquence, autorise de manière inopportune une mise en communication directe des déchets avec l'extérieur lors de leur parcellisation.

Un deuxième inconvénient réside dans la perte de capacité de réception des déchets, en raison de leur réception à l'intérieur de la gaine maintenue tendue au milieu du volume intérieur de la trémie. Par ailleurs, le caractère consommable de la gaine induit des coûts augmentés de fonctionnement du dispositif, d'autant plus importants en raison de la perte de volume susmentionnée.

Les moyens de broyage équipant le compartiment de broyage sont notamment du type à outils tranchants rotatifs. Un problème à résoudre réside dans la désinfection de ces outils tranchants, notamment au moins entre chaque cycle de fonctionnement du dispositif.

Pour cela, le compartiment de broyage est équipé de moyens de désinfection comprenant des buses de projection de vapeur à l'intérieur du compartiment de broyage, axialement suivant l'arbre d'entraînement des outils tranchants rotatifs.

Un premier inconvénient réside dans l'agencement des moyens d'acheminement de la vapeur depuis le générateur jusqu'au buses de projection, qui tendent à induire un colmatage de ces dernières.

Un deuxième inconvénient réside dans décontamination insatisfaisante des outils tranchants.

Un troisième inconvénient réside dans l'organisation générale du compartiment de broyage, qui rend sa maintenance délicate, d'autant qu'il est prévu d'interposer des moyens de séparation entre le compartiment de broyage et le compartiment de désinfection pour interdire une mise en communication avec l'extérieur de ce dernier.

Le compartiment de désinfection des déchets comprend un autoclave pour la réception des déchets broyés. Cet autoclave est équipé de moyens de chauffage pour la décontamination des déchets et d'un plateau de pressage pour pousser les déchets décontaminés vers une porte d'évacuation.

Un premier inconvénient réside dans une désinfection insuffisante des déchets, en raison de leur traitement par chauffage seul et de leur compactage par le plateau de pressage.

D'autres traitements de parcellisetion et de décontamination de déchets sont commus pas les documents WO-A-0038744 et US-A-5213774

Un but de la présente invention est de proposer un dispositif de broyage et de décontamination de déchets médicaux infectieux, agencé pour remédier aux inconvénients susvisés. Plus particulièrement, le but de la présente invention est de proposer un tel dispositif qui soit organisé de manière à permettre un traitement fiable et efficace des déchets, dans un environnement sécurisé.

Le dispositif selon la présente invention est, dans sa généralité, un dispositif de parcellisation et de désinfection ou de traitement de déchets, plus particulièrement adapté aux déchets médicaux infectieux mais pouvant être appliqué à tous autres déchets nécessitant un traitement analogue.

Ce dispositif est, de manière connue en soi, monté sur un bâti et comprend une succession étagée de compartiments, dont un compartiment supérieur d'alimentation en déchets, comprenant une trémie, un compartiment médian de broyage, comprenant des outils tranchants pour parcelliser les déchets, et un compartiment inférieur de désinfection et d'évacuation des résidus.

Selon l'invention,
- le compartiment supérieur comprend des moyens d'acheminement des déchets vers le compartiment médian de broyage, des moyens de confinement du volume intérieur de la trémie entre l'orifice d'admission que celle-ci comprend et son orifice d'évacuation vers le compartiment médian, chacun de ces orifices étant équipés de moyens respectifs d'obturation, manoeuvrables entre une position de dégagement et une position d'obturation de l'orifice correspondant, et de premiers moyens de désinfection des déchets et de la trémie ;
- le compartiment médian débouche directement vers le compartiment inférieur, en étant en communication permanente, à travers un passage, avec ce compartiment inférieur, et comprend des seconds moyens de désinfection des outils tranchants, et
- le compartiment inférieur comprend une cuve de réception des déchets broyés en provenance du compartiment médian, cette cuve étant équipée de troisièmes moyens de désinfection pour le traitement des déchets broyés et de moyens d'évacuation des déchets traités vers une porte d'évacuation.

Ces dispositions sont telles que, pour un cycle donné de traitement des déchets, le compartiment supérieur peut être ouvert sur l'environnement extérieur dans une première étape pour l'introduction des déchets, puis être isolé par la fermeture de l'orifice d'admission de la trémie. L'acheminement des déchets vers le compartiment médian intervient alors, puis, dans une deuxième étape, le broyage et l'introduction des déchets à l'intérieur de la cuve sont effectués en continu, jusqu'à une quantité souhaitée correspondant à celle des déchets préalablement introduits dans la trémie. Enfin, dans une troisième étape, le compartiment supérieur est isolé par une fermeture de l'orifice d'évacuation de la trémie, pour des opérations concomitantes de désinfection des outils et des déchets broyés contenus dans la cuve.

On comprendra également que chacun des compartiments du dispositif est équipé de son propre moyen de désinfection, de sorte que les déchets, au cours de leur progression dans le dispositif, peuvent être désinfectés niveau de chacun des compartiments. Par ailleurs, chaque compartiment, grâce à son moyen de désinfection propre, pourra être désinfecté après chaque cycle de traitement. De même, le compartiment supérieur, avant manoeuvre de son dispositif d'obturation supérieure vers la position de dégagement, pourra être désinfecté. Ainsi, avant toute intervention du personnel au niveau du compartiment supérieur, les faces internes de ce dernier seront désinfectées, ce qui permet réduire les risques de contamination par contact.

On notera aussi que le dispositif selon l'invention est constitué par trois compartiments étanches qui peuvent être désinfectés indépendamment les uns des autres avant ouverture vers l'ambiance extérieure et surtout avant tout démontage et/ou toute intervention du personnel affecté à la maintenance ou à la conduite du dispositif.

Selon une forme préférée de réalisation de l'invention, les moyens d'obturation de l'orifice d'admission de la trémie comprennent un capot articulé à ladite trémie.

Selon une forme préférée de réalisation de l'invention, les moyens d'obturation de l'orifice d'évacuation de la trémie comprennent une vanne d'isolement disposée entre la trémie et le compartiment médian, comportant un corps de vanne solidaire de la trémie d'une part et du compartiment médian d'autre part, ledit corps de vanne comportant une ouverture traversante de passage des déchets et ladite vanne comportant par ailleurs un tiroir monté en coulissement dans le corps de vanne entre une position d'obturation, dans laquelle l'ouverture traversante est obturée et la trémie et le compartiment médian sont isolés l'un de l'autre, et une position de dégagement, dans laquelle l'ouverture traversante est dégagée et la communication entre la trémie le compartiment médian est établie.

Selon une autre caractéristique de l'invention, le corps de vanne, autour de l'ouverture traversante, est équipé d'un joint périphérique gonflable, pour l'étanchéité entre le compartiment supérieur et le compartiment médian, par mise en compression élastique du tiroir coulissant contre le bâti au moyen du joint gonflable, et pour faciliter le coulissement du tiroir par sa libération du joint, hors pression.

Le caractère gonflable de ce joint permet d'obtenir une étanchéité de qualité entre le compartiment supérieur et le compartiment médian, par une mise en compression élastique du tiroir contre le corps de vanne au moyen du joint gonflable. Par ailleurs, le caractère gonflable du joint permet de réduire son volume lorsqu'il est gonflé préalablement à l'ouverture de l'orifice d'évacuation de la trémie, pour faciliter le coulissement du tiroir par sa libération du joint hors pression.

Selon un autre aspect de la présente invention, et compte tenu de l'accès au volume intérieur de la trémie lors de l'opération de broyage, les moyens d'acheminement des déchets depuis le compartiment supérieur vers le compartiment médian sont constitués par un organe de poussée sous forme d'une tige traversant de part en part ledit capot, articulé omnidirectionnellement au capot d'une part et possédant une latitude de déplacement axial limitée d'autre part de manière à lui autoriser un accès à l'ensemble du volume intérieur de la trémie et aux faces internes de celles-ci, ledit organe étant lié de manière étanche audit capot.

Cette articulation omnidirectionnelle et cette latitude de déplacement axial de l'organe de poussée lui autorise un accès à l'ensemble du volume intérieur de la trémie et à ses faces internes, grâce à quoi l'organe de poussée peut diriger les déchets dans leur totalité vers le compartiment médian, y compris ceux adhérant à la face interne de la trémie.

L'organe de poussée peut être lié au capot par un soufflet ou une sphère d'articulation étanche ; il peut également être engagé à coulissement dans une pièce cylindrique reliée à un disque souple, lui-même relié audit capot, cette pièce cylindrique délimitant une chambre interne, traversée par l'organe de poussée, remplie d'un produit bactéricide.

Selon un autre aspect de la présente invention, le compartiment supérieur est équipé de moyens d'admission d'un fluide de traitement (liquide, gaz ou vapeur) comprenant l'un quelconque au moins de premiers moyens d'admission d'un fluide de désinfection pour le traitement de la trémie (liquide, gaz ou vapeur) et de deuxièmes moyens d'admission de liquide de désinfection pour un traitement des déchets par bain.

On relèvera que le traitement par bain des déchets dans le compartiment supérieur est rendu possible grâce à la présence des moyens de confinement de la trémie, qui constituent avantageusement des moyens de rétention du liquide de désinfection pour le traitement des déchets par immersion.

Les premiers moyens d'admission d'un fluide de désinfection (liquide, gaz ou vapeur) pour le traitement de la trémie sont par exemple constitués par au moins une buse de projection du fluide correspondant (liquide, gaz ou vapeur) qui débouche à travers le capot à l'intérieur du volume de la trémie.

Les deuxième moyens d'admission de liquide de désinfection telle que soude, pour un traitement des déchets par bain, son quant à eux réalisés simplement en exploitant la possibilité d'accéder au volume intérieur de la trémie par son orifice d'admission, lorsque le capot est ouvert et que l'orifice d'évacuation de la trémie est obturé, par le tiroir notamment.

Le capot est préférentiellement conformé en dôme, pour favoriser l'écoulement du liquide de désinfection de la trémie sur sa face interne, en vue de son drainage vers le volume intérieur de la trémie.

Selon la présente invention, les outils tranchants sont confinés à l'intérieur d'un châssis agencé en cadre à parois latérales, qui est monté amovible sur le bâti à la manière d'un tiroir. Ces agencement et montage du châssis sont tels qu'ils autorisent une mise en place et, inversement, un retrait faciles des outils, en vue de leur maintenance et/ou de celle du dispositif.

L'intérêt des seconds moyens de désinfection et de permettre le nettoyage et la désinfection des outils avant leur retrait et leur manipulation par l'utilisateur, en cas par exemple d'un dysfonctionnement du dispositif en cours d'un cycle de fonctionnement impliquant son interruption.

Selon une autre caractéristique de l'invention, au moins les seconds et troisièmes moyens de désinfection comprennent un générateur de vapeur chaude commun et chacun un circuit d'injection de cette vapeur chaude dans leur volume interne.

Cette disposition permet une désinfection de ces compartiments au moins par l'action la chaleur. L'intérêt de cette caractéristique est d'amener à température adéquate, avant traitement, le compartiment médian et éventuellement le compartiment inférieur et d'amener à température adéquate les outils tranchants du compartiment médian.

Préférentiellement, de la vapeur d'eau chaude est utilisée pour désinfecter le compartiment médian et le compartiment inférieur, cette vapeur chaude pouvant être additionnée éventuellement d'au moins un agent bactéricide pour renforcer l'action du traitement. Il va toutefois de soi que tout fluide gazeux ou liquide possédant des propriétés bactéricides pourra être utilisé.

Selon une autre caractéristique de l'invention, les outils tranchants sont organisés en deux séries parallèles d'outils tranchants et les outils de chaque série sont montés sur un arbre support d'entraînement en rotation propre à la série d'outils, lesdits outils étant montés sur leurs dits arbres de manière fixe en rotation et translation par rapport à ces derniers. Par ailleurs, les outils de chaque série sont disposés de manière régulièrement espacée les uns des autres de façon qu'entre deux outils consécutifs soit ménagé un intervalle dont la largeur est supérieure à l'épaisseur de chaque outil et les deux séries d'outils sont disposées de manière entrecroisée en ce sens que les outils de chaque série pénètrent dans les intervalles entre les outils de l'autre série. Les deux arbres d'entraînement sont entraînés en rotation en sens inverse l'un de l'autre et selon des vitesses de rotation différentes.

Selon un autre aspect de l'invention, les outils tranchants de chaque série sont séparés les uns des autres par des entretoises engagées sur l'arbre correspondant.

Selon un autre aspect de la présente invention, les deuxièmes moyens de désinfection comprennent au moins un canal d'acheminement d'un fluide désinfectant (liquide, gaz ou vapeur). Ce canal est aménagé à travers au moins l'une des parois latérales du châssis selon une direction radiale aux outils tranchants et se prolonge pour déboucher dans les intervalles entre les outils, de telle sorte que le liquide désinfectant puisse être directement amené depuis l'extérieur du châssis et être projeté contre les faces latérales des outils tranchants suivant une direction de projection contenue dans un plan parallèle à ces faces. On comprendra que ces canaux sont préférentiellement en pluralité en un nombre correspondant à celui des intervalles auxquels ils sont chacun affectés.

Selon un autre aspect de la présente invention, les troisièmes moyens de désinfection équipant la cuve du compartiment inférieur comprennent, pris seuls ou, et de préférence, en combinaison :
a) des moyens de mise sous vide du volume intérieur de la cuve. On notera qu'en raison de la communication permanente entre le compartiment inférieur et le compartiment médian, ce dernier se trouve mis sous vide concomitamment à la mise sous vide de la cuve.
b) des moyens d'admission sous pression d'un fluide désinfectant à l'intérieur de cette cuve, tels que vapeur d'eau accessoirement mêlée d'un agent traitant, mais il sera possible d'utiliser un liquide ou un gaz désinfectant.
c) des moyens de brassage des déchets broyés, pour leur malaxage pendant la phase de désinfection.

Ces moyens de brassage sont avantageusement constitués par une vis sans fin manoeuvrable alternativement suivants deux sens de rotation, pour permettre d'adapter le temps de l'opération de malaxage selon la délivrance de déchets broyés en provenance du compartiment médian. Par ailleurs, cette vis sans fin est avantageusement exploitée pour constituer les moyens d'évacuation des déchets broyés de manière à les acheminer vers la porte d'évacuation, à partir d'une rotation continue de la vis sans fin dans le sens correspondant.

On remarquera que les moyens d'admission du fluide désinfectant (gaz ou liquide ou vapeur) à l'intérieur de la cuve sont avantageusement constitués par des buses ménagées dans l'arbre de rotation de la vis sans fin et occupant une position radiale par rapport à cet arbre, ces buses étant alimentées en fluide depuis l'extérieur, au moyen d'un canal s'étendant axialement à l'intérieur de l'arbre de rotation de la vis sans fin.

Par ailleurs, une grille d'essorage des déchets traités est avantageusement disposée en sortie de cuve, et le dispositif comprend en outre des moyens d'évacuation des exsudats, disposés à la base de la cuve.

D'autres buts, avantages et caractéristiques de la présente invention apparaîtront, à la lecture de la description d'une forme préférée de réalisation donnée à titre d'exemple non limitatif, en se référant aux figures de dessins ci-annexées, en lesquelles :
les figures 1 à 10 illustrent schématiquement un exemple de réalisation du dispositif selon l'invention, à diverses étapes successives de sa mise en oeuvre ;
la figure 11 est une vue schématique, en coupe, du dispositif illustré sur les figures précédentes ;
la figure 12 est une vue schématique, en coupe, d'un châssis porteur des outils tranchants équipant un dispositif illustré sur les figures précédentes ;
les figures 13 et 14 sont des vues en coupe d'un détail, selon une variante de réalisation.

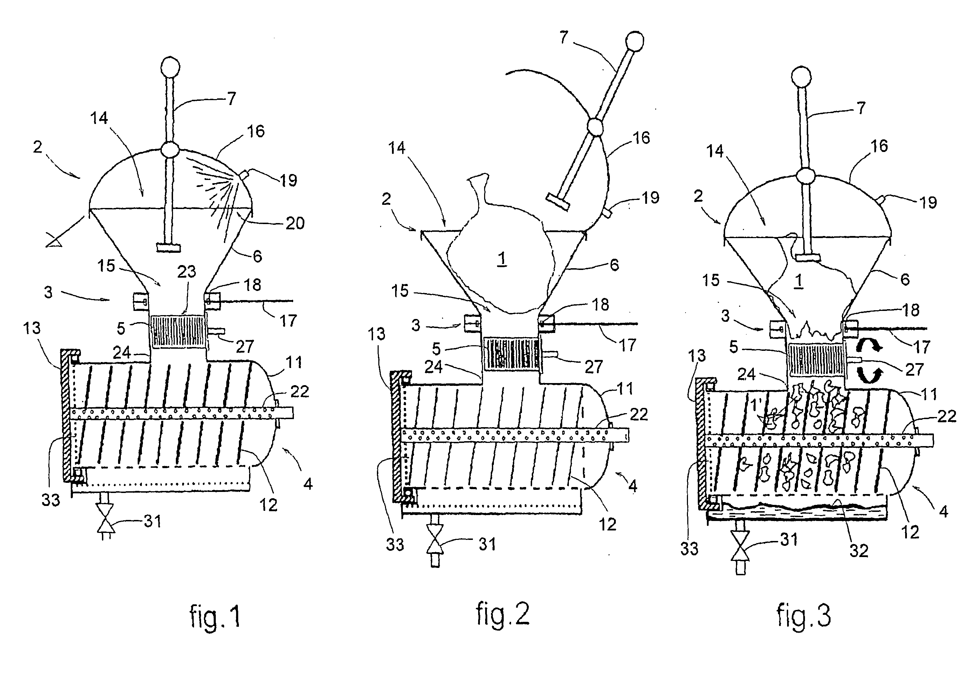

Sur les figures 1 à 11, un dispositif selon la présente invention est représenté, comprenant principalement trois compartiments 2, 3, 4 en succession étagée, dont un compartiment supérieur 2 d'alimentation en déchets, un compartiment médian 3 de broyage des déchets et un compartiment inférieur 4 de traitement des déchets broyés.

Le compartiment supérieur 2 d'alimentation en déchets 1 est principalement constitué d'une trémie 6 surmontée d'un capot 16. L'orifice inférieur de la trémie 6 est obturable au moyen d'une vanne d'isolement disposée entre la trémie 6 et le compartiment médian 3, comportant un corps de vanne solidaire de la trémie d'une part et du compartiment médian d'autre part, ledit corps de vanne comportant une ouverture traversante de passage des déchets. Ladite vanne comporte par ailleurs un tiroir 17 monté en coulissement dans le corps de vanne entre une position d'obturation, dans laquelle l'ouverture traversante est obturée et la trémie et le compartiment médian sont isolés l'un de l'autre, et une position de dégagement, dans laquelle l'ouverture traversante est dégagée et la communication entre la trémie et le compartiment médian est établie.

Le corps de vanne autour de l'ouverture traversante est équipé d'un joint périphérique 18 gonflable, pour l'étanchéité entre le compartiment supérieur 2 et le compartiment médian 3, par une mise en compression élastique du tiroir coulissant 17 contre le bâti 24 au moyen du joint gonflable 18, et pour faciliter le coulissement du tiroir 17 par sa libération du joint 18, hors pression.

Un organe de poussée 7, sous forme de tige, traverse de part en part le capot 16 et est articulé omnidirectionnellement et de manière étanche audit capot. Cette articulation est réalisée au moyen d'une sphère recevant l'organe 7 à coulissement, montée pivotante dans un siège solidaire du capot. Cet organe de poussée 7 possède, par son coulissement par rapport à ladite sphère, une latitude de déplacement axial limitée, dans un sens et dans l'autre, de sorte que son extrémité interne à la trémie 6 peut accéder à l'ensemble du volume intérieur de cette trémie 6 et aux faces internes de celle-ci.

Une buse 19 de projection de fluide de désinfection 20 (liquide, gaz ou vapeur) dans la trémie 6 débouche à travers le capot 16. On notera que ce dernier est conformé en dôme et est avantageusement transparent pour autoriser un accès visuel aux déchets 1 lors de leur acheminement vers le compartiment médian 3.

Le compartiment médian 3 est réservé au broyage des déchets 1 au moyen d'outils tranchants 5 rotatifs logés à l'intérieur d'un châssis 23 amovible. Ces outils tranchants 5 rotatifs sont entraînés en rotation depuis l'extérieur du compartiment médian 3 par l'intermédiaire de deux arbres 27 qui les supportent, et qui émergent hors du châssis 23.

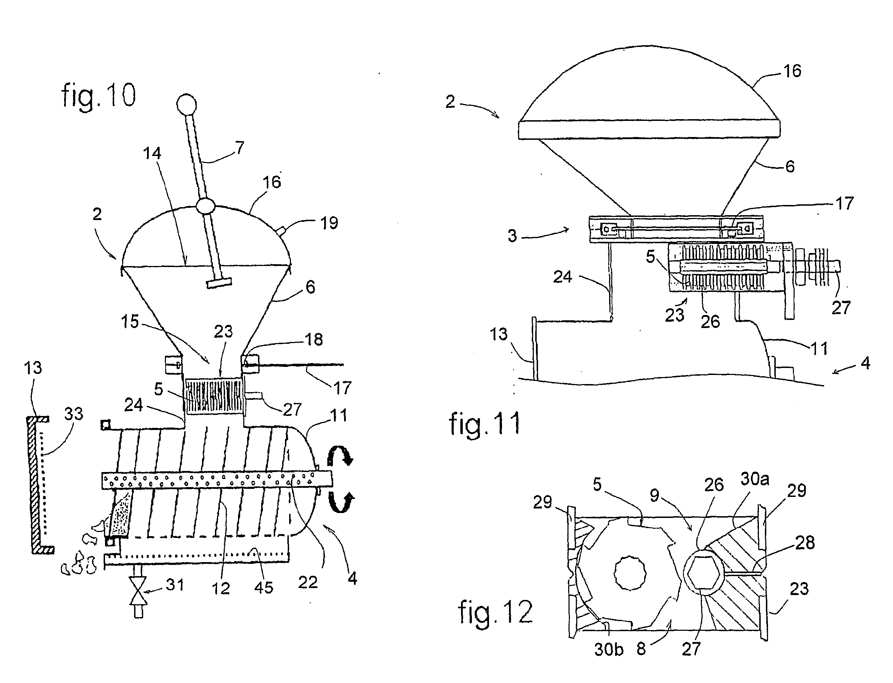

Sur la figure 11, le compartiment médian 3 est principalement constitué d'un châssis 23 supporté par le bâti du dispositif de manière amovible. Plus particulièrement, ce châssis 23 est organisé en tiroir extractible contenant les outils tranchants 5 rotatifs, qui sont répartis en un couple de pluralité d'outils tranchants 5 entretoisés, sur des arbres respectifs 27 de rotation, qui sont entraînées depuis l'extérieur du châssis 23. On remarquera que le châssis 23, conformé en cadre, offre un passage 8 à sa partie inférieure pour le liquide désinfectant 21 en provenance du compartiment inférieur 4, et un passage 9 à sa partie supérieure, pour l'introduction des déchets 1.

Les arbres 27 sont guidés en rotation par des paliers solidaires du châssis 23 du compartiment médian 3.

Les outils tranchants 5 comportent chacun deux ou plusieurs lames radiales de coupe et sont pourvus chacun d'un alésage traversant polygonal par lesquels ils sont engagés sur leur arbre 27 respectif, ce dernier présentant une section droite polygonale pour assurer, en combinaison avec la forme polygonale de l'alésage de chaque outil tranchant, la liaison en rotation de chaque outil tranchant sur ledit arbre.

Les outils tranchants 5 sont maintenus à écartement constant les uns des autres par des entretoises 26 engagées sur leurs arbres respectifs 27, l'épaisseur de chaque entretoise 26 étant supérieure à l'épaisseur de chaque outil tranchant 5, de façon que les outils tranchants portés par un arbre puissent venir dans les intervalles entre les outils tranchants portés par l'autre arbre. Deux séries d'outils tranchants sont ainsi formées, en relation d'interpénétration mutuelle.

Selon la forme préférée de réalisation de l'invention, l'épaisseur des entretoises 26 est supérieure de quelques dixièmes de millimètre à celle des outils tranchants.

Ainsi, l'écart entre les outils tranchants 5 d'une série et les outils tranchants 5 de l'autre série, mesurée entre l'une des faces planes d'un outil d'une série et la face plane contiguë d'un outil dé l'autre série, est très faible et n'est pas suffisant pour le passage de petits objets comme des épingles, des aiguilles et autres. Par ailleurs, l'enveloppe géométrique circulaire associés à chaque outil tranchant 5 (trajectoire circulaire de plus grand diamètre parcourue par l'extrémité de chaque dent des outils tranchants) est écartée de la face cylindrique de l'entretoise 26 en vis-à-vis située sur l'autre arbre, de quelques dixièmes de mm, afin de pouvoir happer et sectionner des petits objets comme des aiguilles et autres. Les deux arbres sont entraînés en rotation en sens inverse l'un de l'autre de façon que les déchets soient happés en partie supérieure et rejetés en partie inférieure. Avantageusement, ils sont entraînés selon des vitesses de rotation différentes de façon à superposer à l'effet de tranchage un effet d'étirement, qui améliore le fractionnement de ces déchets.

Sur la figure 12, il apparaît que l'une des parois latérales du châssis 23 comporte le circuit de distribution du fluide désinfectant formé par des canaux 28 d'acheminement dudit fluide depuis l'extérieur du dispositif, traversant ladite paroi selon une direction radiale aux outils tranchants, vers les faces latérales des outils tranchants 5 suivant une direction de projection contenue dans un plan parallèle à ces faces.

Préférentiellement, chaque paroi latérale comporte un élément de flanc 29 parallèle aux arbres d'entraînement 27 auxquels est associé un empilement d'éléments de paroi 30a, 30b, chaque élément de paroi 30a, 30b s'étendant de matière perpendiculaire aux arbres d'entraînement 27.

Préférentiellement, comme le montre la figure 12, deux types d'éléments de paroi 30a, 30b sont prévus sur les deux parois latérales opposées parallèles aux arbres 27, ces éléments 30a, 30b étant disposés en alternance. Les éléments 30a du premier type pénètrent dans les intervalles entre les outils tranchants 5 et comportent chacun une échancrure en arc de circonférence de cercle pour venir épouser les entretoises 26 et venir au plus près de ces dernières, et les éléments 30b du second type possèdent également chacun une échancrure en arc de circonférence de cercle et sont amenés par ces échancrures au plus près de la trajectoire de l'extrémité des dents des outils tranchants 5.

Préférentiellement, chaque élément 30a, 30b possède une forme de tenon par laquelle il s'engage dans une forme de mortaise pratiquée dans l'élément de flanc 29.

Le circuit de distribution du fluide de traitement est formé par un premier canal longitudinal formé dans les tenons et mortaises et par des canaux transversaux 28 creusés dans les faces latérales des éléments de paroi 30a, 30b et ce de manière radiale aux arbres d'entraînement 27.

Ces canaux 28 sont formés par des rainures et ces dernières viennent en regard des faces planes des outils tranchants 5.

Le compartiment inférieur 4 de traitement des déchets broyés 1' comprend une cuve 11 pour la réception de ces déchets, une vis sans fin 12 pour leur malaxage et leur acheminement vers une porte 13 d'évacuation, et une pompe à vide reliée au volume délimité par la cuve 11, de mise sous vide de ce volume.

La vis 12 comprend un arbre creux présentant des trous radiaux 22 et est reliée à une source de production de vapeur. Elle permet ainsi d'introduire de la vapeur à l'intérieur de la cuve 11, qui constitue un fluide de désinfection des déchets broyés 1 ", et forme des moyens de brassage de ces déchets broyés 1 ", pour leur malaxage pendant la phase de désinfection. Cette vis 12 forme en outre des moyens d'évacuation de ces déchets broyés 1" vers la porte 13.

On remarquera la présence avantageuse d'une grille 33 d'essorage placée directement en amont de cette porte 13 d'évacuation. On relèvera aussi la présence de moyens 31 d'évacuation des exsudats 32, ces moyens comprenant une vanne de vidange notamment.

Après traitement, les déchets pourront être compactés dans le compartiment inférieur par l'action de poussée exercée par la vis sans fin 12 sur ces derniers ; la porte étant fermée, les déchets sont donc comprimés entre la vis 12 et la porte 13 afin de réduire leur volume avant évacuation.

Les figures 1 à 10 illustrent successivement les étapes d'exécution d'un cycle de traitement des déchets, comprenant :
Figure 1 : désinfection du compartiment supérieur 2 par projection du liquide de désinfection 20, avant ouverture du capot 16 d'obturation de l'orifice 14 d'admission en déchets 1 de la trémie 6 ;
Figure 2 : ouverture du capot 16 et introduction des déchets 1 dans la trémie 6, puis fermeture du capot 16 ;
Figure 3: acheminement des déchets au moyen de l'organe de poussée 7 depuis la trémie 6 vers les outils tranchants 5 pour leur broyage, et introduction des déchets broyés 1' à l'intérieur de la cuve 11 ;

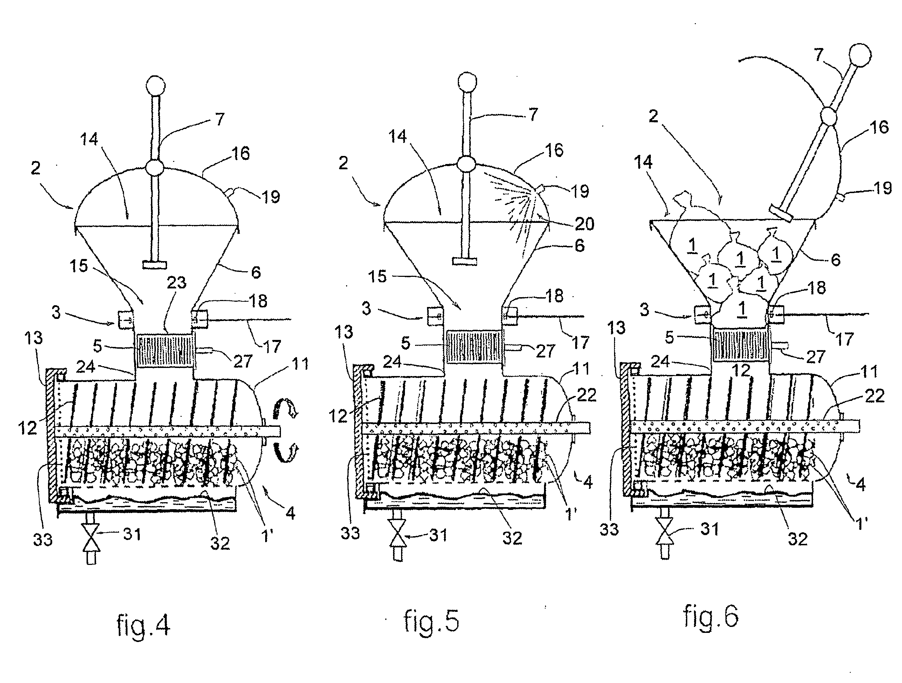

Figure 4 : malaxage des déchets broyés 1' à l'intérieur de la cuve 11, à partir d'une rotation de la vis sans fin 12 alternativement dans les deux sens ;

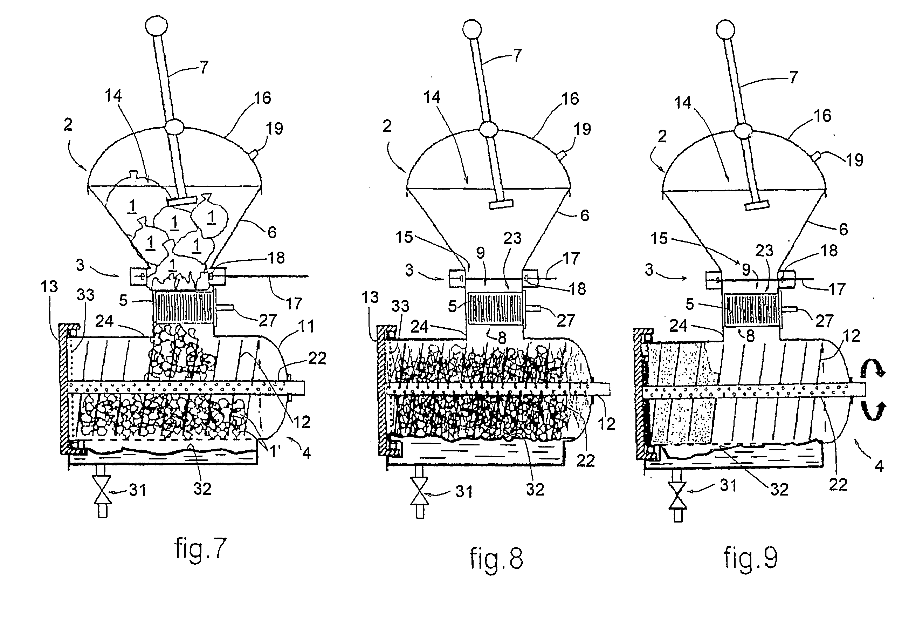

Figures 5, 6 et 7 : dans le cas d'une introduction à l'intérieur de la trémie 6 d'une nouvelle quantité de déchets 1, désinfection éventuelle du compartiment supérieur 2 par projection du liquide de désinfection 20 (figure 5), puis introduction de déchets 1 (figure 6) et poussée de ces déchets 1 vers le compartiment médian 3 (figure 7) ;
Figure 8 : fermeture du tiroir coulissant 17 pour isoler la trémie 6 et les compartiments médian 3 et inférieur 4, malaxage des déchets broyés 1', mise sous vide du volume intérieur de la cuve 11 et projection de liquide de désinfection 20, pour la désinfection des déchets broyés 1' et des outils tranchants 5, par circulation à travers un passage de communication 8, ménagé entre les compartiments médian 3 et inférieur 4 ;
Figure 9: mise à l'atmosphère du volume intérieur de la cuve 11, essorage contre la grille 33 et acheminement des déchets traités 1" vers la porte 13 d'évacuation, puis vidange des exsudats 32 par rotation de la vis sans fin 12 dans le sens correspondant ;
Figure 10 : ouverture de la porte 13 et évacuation des déchets traités 1" hors de la cuve 11.

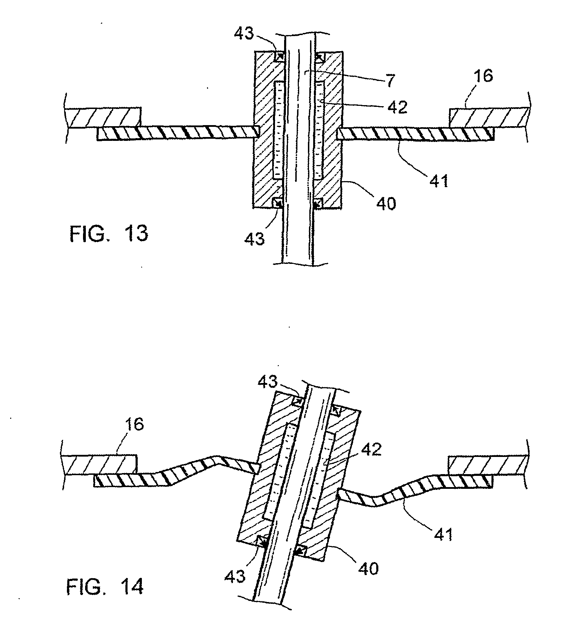

Les figures 13 et 14 représentent une variante de réalisation de l'articulation de l'organe de poussée 7 au capot 16. Dans ce cas, l'organe 7 est engagé à coulissement dans une pièce cylindrique 40 reliée à un disque 41 en élastomère de silicone, lui-même relié au capot 16, par collage. Cette pièce cylindrique 40 délimite une chambre interne 42, traversée par l'organe de poussée 7, remplie d'un gel bactéricide et est fermée à ses extrémités par des joints à lèvre 43.

On relèvera que les mises en oeuvre successives des différents moyens du dispositif selon l'invention peuvent être avantageusement commandées à partir d'un automate.

Il va de soi que la présente invention peut recevoir tous aménagements et variantes du domaine des équivalents techniques sans pour autant sortir du cadre du présent brevet. Ainsi, l'injection de fluide (vapeur) de désinfection dans la cuve 11 peut être réalisée par un autre moyen que par les trous 22, en particulier en partie basse de cette cuve 11, au travers d'une grille 45 de filtration des exsudats 32 que peut comprendre la cuve 11 (cf. figure 10).

## Revendications

1. Dispositif de parcellisation et de décontamination ou de traitement de déchets (1), notamment de déchets médicaux infectieux, monté sur un bâti (24) et comprenant une succession étagée de compartiments (2, 3, 4), dont un compartiment supérieur (2) d'alimentation en déchets, comprenant une trémie (6), un compartiment médian (3) de broyage, comprenant des outils tranchants (5) pour parcelliser les déchets, et un compartiment inférieur (4) de désinfection et d'évacuation des résidus ;
dispositif **caractérisé en ce que :**
- le compartiment supérieur (2) comprend des moyens d'acheminement (7) des déchets vers le compartiment médian (3) de broyage, des moyens de confinement du volume intérieur de la trémie (6) entre l'orifice d'admission (14) que celle-ci comprend et son orifice (15) d'évacuation vers le compartiment médian (3), chacun de ces orifices (14, 15) étant équipés de moyens respectifs d'obturation (16, 17), manoeuvrables entre une position de dégagement et une position d'obturation de l'orifice correspondant, et de premiers moyens (19) de désinfection des déchets (1) et de la trémie (6) ;
- le compartiment médian (3) débouche directement vers le compartiment inférieur (4), en étant en communication permanente, à travers un passage (8), avec ce compartiment inférieur (4), et comprend des seconds moyens de désinfection des outils tranchants (5) ;
- les outils tranchants (5) sont confinés à l'intérieur d'un châssis (23) agencé en cadre à parois latérales, monté amovible sur le bâti (24) à la manière d'un tiroir, délimitant le passage (8) et constituent principalement le compartiment médian (3), et
- le compartiment inférieur (4) comprend une cuve (11) de réception des déchets broyés en provenance du compartiment médian (3), cette cuve (11) étant équipée de troisièmes moyens (21, 22) de désinfection, pour le traitement des déchets broyés et la désinfection des outils tranchants (5) au travers du passage (8), et de moyens (12) d'évacuation des déchets traités vers une porte d'évacuation (13).

2. Dispositif selon la revendication 1, **caractérisé en ce que** les moyens d'obturation de l'orifice d'admission (14) de la trémie (6) comprennent un capot (16) articulé à ladite trémie (6).

3. Dispositif selon la revendication 1 ou la revendication 2, **caractérisé en ce que** les moyens d'obturation de l'orifice d'évacuation (15) de la trémie (6) comprennent une vanne d'isolement disposée entre la trémie (6) et le compartiment médian (3), comportant un corps de vanne solidaire de la trémie (6) d'une part et du compartiment médian (3) d'autre part, ledit corps de vanne comportant une ouverture traversante de passage des déchets et ladite vanne comportant par ailleurs un tiroir (17) monté en coulissement dans le corps de vanne entre une position d'obturation, dans laquelle l'ouverture traversante est obturée et la trémie (6) et le compartiment médian (3) sont isolés l'un de l'autre, et une position de dégagement, dans laquelle l'ouverture traversante est dégagée et la communication entre la trémie (6) le compartiment médian (3) est établie.

4. Dispositif selon la revendication 3, **caractérisé en ce que** le corps de vanne, autour de l'ouverture traversante, est équipé d'un joint périphérique gonflable (18) pour l'étanchéité entre le compartiment supérieur (2) et le compartiment médian (3), par mise en compression élastique du tiroir (17) coulissant contre le bâti (24) au moyen du joint gonflable (18), et pour faciliter le coulissement du tiroir (17) par sa libération du joint (18), hors pression:

5. Dispositif selon l'une des revendications 2 à 4, **caractérisé en ce que** les moyens d'acheminement des déchets depuis le compartiment supérieur (2) vers le compartiment médian (3) sont constitués par un organe (7) de poussée sous forme d'une tige traversant de part en part ledit capot (16), articulé omnidirectionnellement au capot (16) d'une part et possédant une latitude de déplacement axial limitée d'autre part de manière à lui autoriser un accès à l'ensemble du volume intérieur de la trémie (6) et aux faces internes de celles-ci, ledit organe étant lié de manière étanche audit capot (16).

6. Dispositif selon la revendication 5, **caractérisé en ce que** l'organe de poussée (7) est engagé à coulissement dans une pièce cylindrique (40) reliée à un disque souple (41), lui-même relié audit capot (16), cette pièce cylindrique (40) délimitant une chambre interne (42), traversée par l'organe de poussée (7), remplie d'un produit bactéricide.

7. Dispositif selon l'une des revendications 1 à 6, **caractérisé en ce que** le compartiment supérieur (2) est équipé de moyens d'admission d'un fluide de traitement (liquide, gaz ou vapeur) comprenant l'un quelconque au moins de premiers moyens (19) d'admission d'un fluide de désinfection (20) pour le traitement de la trémie (6) (liquide, gaz ou vapeur) et de deuxièmes moyens d'admission de liquide de désinfection pour un traitement des déchets (1) par bain.

8. Dispositif selon la revendication 7, **caractérisé en ce que** les moyens (16, 17) de confinement de la trémie (6) constituent des moyens de rétention du liquide de désinfection pour le traitement des déchets (1) par immersion.

9. Dispositif selon les revendications 2 et 7, **caractérisé en ce que** les premiers moyens d'admission d'un fluide de désinfection (20) (liquide, gaz ou vapeur) pour le traitement de la trémie (6) sont constitués par au moins une buse (19) de projection du fluide correspondant (liquide, gaz ou vapeur) qui débouche à travers le capot (16) à l'intérieur du volume de la trémie (6).

10. Dispositif selon l'une des revendications 2 à 9, **caractérisé en ce que** le capot (16) est conformé en dôme, pour favoriser l'écoulement du liquide de désinfection (20) de la trémie (6) sur sa face interne, en vue de son drainage vers le volume intérieur de la trémie (6).

11. Dispositif selon l'une des revendications 1 à 9, **caractérisé en ce que** les outils tranchants (5) sont organisés en deux séries parallèles d'outils tranchants (5), les outils de chaque série étant montés sur un arbre support (27) d'entraînement en rotation propre à la série d'outils, lesdits outils étant montés sur leurs dits arbres (27) de manière fixe en rotation et translation par rapport à ces derniers, les outils de chaque série étant disposés de manière régulièrement espacée les uns des autres de façon qu'entre deux outils consécutifs soit ménagé un intervalle dont la largeur est supérieure à l'épaisseur de chaque outil, et les deux séries d'outils étant disposées de manière entrecroisée en ce sens que les outils de chaque série pénètrent dans les intervalles entre les outils de l'autre série.

12. Dispositif selon la revendication 11, **caractérisé en ce que** les deux arbres (27) d'entraînement sont entraînés en rotation depuis l'extérieur du bâti (24), en sens inverse l'un de l'autre et selon des vitesses de rotation différentes.

13. Dispositif selon la revendication 11 ou la revendication 12, **caractérisé en ce que** les outils tranchants (5) de chaque série sont séparés les uns des autres par des entretoises (26) engagées sur l'arbre correspondant.

14. Dispositif selon l'une des revendications 11 à 13, **caractérisé en ce que** les deuxièmes moyens de désinfection comprennent au moins un canal (28) d'acheminement d'un fluide désinfectant (liquide, gaz ou vapeur); qui est aménagé à travers au moins l'une des parois latérales du châssis (23) selon une direction radiale aux outils tranchants (5) et se prolonge pour déboucher dans les intervalles entre les outils, de telle sorte que le liquide désinfectant puisse être directement amené depuis l'extérieur du châssis (23) et être projeté contre les faces latérales des outils tranchants (5) suivant une direction de projection contenue dans un plan parallèle à ces faces.

15. Dispositif selon l'une des revendications 1 à 14, **caractérisé en ce que** les troisièmes moyens de désinfection équipant la cuve (11) du compartiment inférieur (4) comprennent des moyens de mise sous vide du volume intérieur de la cuve (11).

16. Dispositif selon l'une des revendications 1 à 15, **caractérisé en ce que** les troisièmes moyens de désinfection (22) équipant la cuve (11) du compartiment inférieur (4) comprennent des moyens d'admission sous pression d'un fluide désinfectant (21) à l'intérieur de cette cuve (11).

17. Dispositif selon l'une des revendications 1 à 16, **caractérisé en ce que** les troisièmes moyens de désinfection équipant la cuve (11) du compartiment inférieur (4) sont compris dans des moyens (12) de brassage des déchets broyés (1'), pour leur malaxage pendant la phase de désinfection.

18. Dispositif selon la revendication 17, **caractérisé en ce que** les moyens de brassage sont constitués par une vis sans fin (12) manoeuvrable alternativement suivants deux sens de rotation, pour permettre d'adapter le temps de l'opération de malaxage selon la délivrance de déchets broyés (1') en provenance du compartiment médian (3).

19. Dispositif selon la revendication 18, **caractérisé en ce que** les moyens d'évacuation des déchets broyés (1') sont constitués par la vis sans fin (12) pour leur acheminement vers la porte d'évacuation (13), à partir d'une rotation continue de la vis sans fin (12) dans le sens correspondant.

20. Dispositif selon l'une des revendications 1 à 19, **caractérisé en ce qu'**il comprend une grille (33) d'essorage des déchets traités (1") disposée en sortie de cuve (11) et des moyens (31) d'évacuation des exsudats (32), disposés à la base de la cuve (11).

21. Méthode pour le traitement de déchets (1) par parcellisation et désinfection mettant en oeuvre un dispositif selon la revendication 1, **caractérisée en ce que**, pour un cycle donné de traitement des déchets (1), elle consiste à effectuer principalement les étapes suivantes :
- ouverture du compartiment supérieur (2) sur l'environnement extérieur pour l'introduction des déchets (1) puis fermeture de l'orifice d'admission (14) de la trémie (6) et acheminement des déchets (1) vers le compartiment médian (3) ;
- broyage et introduction en continu des déchets broyés (1') à l'intérieur de la cuve (11) jusqu'à une quantité souhaitée, correspondante à celle des déchets (1) préalablement introduits dans la trémie (6) ;
- isolement du compartiment supérieur (2) par fermeture de l'orifice d'évacuation (15)de la trémie (6)
- opérations concomitantes de désinfection des outils et des déchets broyés (1') contenus dans la cuve (11).

22. Méthode selon la revendication 21, mettant en oeuvre un dispositif selon les revendications 1 à 19, **caractérisée en ce qu'**elle consiste plus particulièrement à opérer les étapes d'exécution d'un cycle de traitement déchets (1), comprenant les étapes successives suivantes :
a) désinfection du compartiment supérieur (2) par projection de liquide de désinfection, avant ouverture du capot (16) d'obturation de l'orifice d'admission (14) des déchets (1) de la trémie (6) ;
b) ouverture du capot (16) et introduction des déchets (1) dans la trémie (6), puis fermeture capot (16) ;
c) acheminement des déchets (1) au moyen de l'organe de poussée depuis la trémie (6) vers les outils tranchants pour leur broyage et introduction des déchets broyés (1') à l'intérieur de la cuve (11) ;
d) malaxage des déchets broyés (1') à l'intérieur la cuve (11), à partir d'une rotation de la vis sans fin (12) alternativement dans les deux sens ;
e) dans le cas d'une introduction à l'intérieur de la trémie (6) d'une nouvelle quantité de déchets (1), désinfection du compartiment supérieur (2) par projection d'un liquide de désinfection puis introduction de déchets (1) et poussée de ces déchets (1) vers le compartiment médian (3) ;
f) fermeture du tiroir (17) coulissant pour isoler la trémie (6) des compartiment médian (3) et inférieur puits malaxage des déchets broyés (1') ;
g) mise sous vide du volume intérieur de la cuve (11) et projection d'un liquide désinfectant pour la désinfection déchets broyés (1') et des outils tranchants (5) à partir d'une circulation du liquide désinfectant à travers le passage de communication ménagé entre les compartiments médian et inférieur ;
h) mise à l'atmosphère du volume intérieur de la cuve (11), essorage des déchets (1) contre la grille et acheminement des déchets traités (1") vers la porte d'évacuation (13), puis vidange des exsudats (32) par rotation de la vis sans fin (12) dans le sens correspondant ;
i) ouverture de la porte d'évacuation (13) et évacuation hors de la cuve (11) des déchets traités (1").

## Claims

1. Device for the breakdown and disinfection or treatment of waste, in particular of infectious medical waste (1), mounted on a framework (24) and comprising a successive series of compartments (2, 3, 4), including an upper compartment feeding waste (2), equipped with a hopper (6), an intermediate shredding compartment (3), including cutting tools (5) to break down the waste, and a lower compartment (4) for disinfecting and evacuating residues;
device **characterized in that:**
- the upper compartment comprises means (7) for conveying waste towards the intermediate shredding compartment (3), means for confining the internal volume of the hopper (6) between the intake orifice (14) comprised therein and its discharge orifice (15) towards the intermediate compartment (3), each of these orifices (14, 15) being equipped with respective closure means (16, 17), able to be maneuvered between a disengaged position and a closed position of the corresponding orifice, and first means (19) for disinfecting the waste (1) and the hopper (6);
- the intermediate compartment (3) emerges directly towards the lower compartment (4), by being in permanent communication, through a passage (8), with said lower compartment (4), and includes second means for disinfecting the cutting tools (5);
- the cutting tools (5) are confined to the inside of a frame (23) arranged in a framework of lateral walls, removably mounted on the framework (24) like a slide valve, delimiting the passage (8) and mainly constituting the intermediate compartment (3), and
- the lower compartment (4) includes a vessel (11) for receiving shredded waste from the intermediate compartment (3), said vessel (11) being equipped with third disinfecting means (21, 22) for treating the shredded waste and means (12) for evacuating treated waste through an evacuation door (13).

2. Device according to claim 1, **characterized in that** the closing means of the intake orifice (14) of the hopper (6) comprise a cover (16) articulated to said hopper (6).

3. Device according to claim 1 or claim 2, **characterized in that** the closure means of the discharge orifice (15) of the hopper (6) comprise an isolation valve arranged between the hopper (6) and the intermediate compartment (3), comprising a valve body integral with the hopper (6) on one hand and the intermediate compartment (3) on the other, said valve body comprising a through opening for the passage of waste and said valve also comprising a slide valve (17) slidingly mounted in the valve body between a closed position, in which the through opening is closed and the hopper (6) and intermediate compartment (3) are isolated from each other, and a disengaged position, in which the through opening is disengaged and communication between the hopper (6) and the intermediate compartment (3) is established.

4. Device according to claim 3, **characterized in that** the valve body, around the through opening, is equipped with an inflatable peripheral seal (18), for sealing between the upper compartment (2) and the intermediate compartment (3), by elastic compression of the slide valve (17) sliding against the framework (24) through an inflatable seal (18), and to facilitate sliding of the slide valve (17) by freeing it from the seal (18), pressure-free.

5. Device according to anyone of claims 2 to 4, **characterized in that** the means for conveying waste from the upper compartment (2) towards the intermediate compartment (3) are made up of a driving member (7) in the form of a rod going straight through said cover (16), articulated omni-directionally to the cover (16) on one hand and having a limited axial movable range on the other hand, so as to allow it access to the entirety of the internal volume of the hopper (6) and to the internal surfaces thereof, said member being sealably connected to said cover (6).

6. Device according to claim 5, **characterized in that** the driving member (7) is slidingly engaged in a cylindrical part (40) connected to a flexible disk (41), itself connected to said cover (16), this cylindrical part (40) defining an internal chamber (42), crossed by the driving member (7), filled with a bactericidal product.

7. Device according to anyone of claims 1 to 6, **characterized in that** the upper compartment (2) is equipped with means for admitting a treatment fluid, such as a liquid, gas or vapor, comprising at least any one of first means (19) for admitting a disinfecting fluid (20) for treatment of the hopper (6), such as a liquid, gas or vapor, and second means for admitting a disinfecting liquid for bath treatment of the waste (1).

8. Device according to claim 7, **characterized in that** the means (16, 17)) for confining the hopper (6) constitute means for holding the disinfection liquid for waste treatment by immersion.

9. Device according to claims 2 and 7, **characterized in that** the first means for admitting a disinfecting fluid (20), such as a liquid, gas or vapor, for treatment of the hopper (6) are made up of at least one projection nozzle (19) for a corresponding fluid (liquid, gas or vapor) which emerges through the cover (16) inside the volume of the hopper (6).

10. Device according to anyone of claims 2 to 9, **characterized in that** the cover (16) is formed in a dome, to promote the flow of the disinfecting fluid (20) of the hopper (6) on its internal surface, with a view to its draining towards the internal volume of the hopper (6).

11. Device according to anyone of claims 1 to 9, **characterized in that** the cutting tools (5) are organized in two parallel series of cutting tools (5) and the tools (5) of each series are mounted on a support shaft (27) for rotational driving unique to the series of tools, said tools being mounted on said shafts (27) so as to be fixed in rotation and translation relative to the latter, the tools of each series being arranged regularly spaced from each other such that between two consecutive tools there is an interval, the width of which is greater than the thickness of each tool, and the two series of tools being arranged such that they are intertwined in the sense that the tools from each series penetrate the intervals between the tools from the other series.

12. Device according to claim 11, **characterized in that** the two drive shafts (27) are driven in rotation from the outside of the framework (24), in opposite directions relative to each other and at different rotational speeds.

13. Device according to claim 11 or claim 12, **characterized in that** the cutting tools (5) of each series are separated from each other by spacers (26) engaged on the corresponding shaft.

14. Device according to anyone of claims 11 to 13, **characterized in that** the second disinfection means comprise at least one channel (28) for conveying a disinfecting fluid (liquid, gas or vapor), which is arranged through at least one of the side walls of the frame (23) following a direction radial to the cutting tools (5) and extends to emerge in the intervals between the tools, such that the disinfecting liquid can be directly brought from the outside of the frame (23) and projected against the lateral surfaces of the cutting tools (5) along a projection direction contained in a plane parallel to these surfaces.

15. Device according to anyone of claims 1 to 14, **characterized in that** the third disinfection means equipping the vessel (11) of the lower compartment (4) comprise means for evacuating the internal volume of the vessel (11).

16. Device according to anyone of claims 1 to 15, **characterized in that** the third disinfection means (22) equipping the vessel (11) of the lower compartment (4) comprise means for pressurized admission of a disinfecting fluid (21) inside this vessel (11).

17. Device according to anyone of claims 1 to 16, **characterized in that** the third disinfection means equipping the vessel (11) of the lower compartment (4) are comprised in means (12) for mixing the shredded waste (1'), to mix it during the disinfection phase.

18. Device according to claim 17, **characterized in that** the mixing means are made up of a worm (12) able to be alternatingly maneuvered in two rotational directions, to allow one to adapt the mixing operation time according to the delivery of shredded waste from the intermediate compartment (3).

19. Device according to claim 18, **characterized in that** the means for evacuating the shredded waste (1') are made up of the worm (12) conveying it towards the evacuation door (13), from a continuous rotation of the worm (12) in the corresponding direction.

20. Device according to anyone of claims 1 to 19, **characterized in that** it comprises a drain grid (33) for the treated waste (1") arranged at the outlet of the vessel (11), and means (31) for evacuating exudates (32), arranged at the base of the vessel (1).

21. Method for the treatment of waste (1) by breakdown and disinfection using the device according to claim 1, **characterized in that**, for a given waste treatment cycle (1), it includes the following steps:
- opening of the upper compartment (2) to the external environment for the introduction of waste (1), then isolated by closing the intake orifice (14) of the hopper (6) and conveying of the waste (1) towards the intermediate compartment (3)
- continuous shredding and introduction of the shredded waste (1') inside the vessel (11), up to a desired quantity corresponding to that of the waste (1) previously introduced into the hopper (6);
- isolating the upper compartment (2) by closing the discharge orifice (15) of the hopper (6);
- simultaneous operations to disinfect the tools and the shredded waste (1') contained in the vessel (11).

22. Method according to claim 21, using a device according to the claims 1 to 19, **characterized in that** it consist in carrying out the steps of a waste treatment cycle, comprising the following steps:
a) disinfection of the upper compartment (2) by projecting the disinfecting liquid, before opening the cover (16) closing the intake orifice (14) of the hopper (6) for waste (1);
b) opening the cover (16) and introduction of waste (1) in the hopper (6), then closing of the cover (16);
c) conveying waste (1) through a driving member from the hopper (6) towards the cutting tools for its shredding, and introduction of shredded waste (1') inside the vessel (11);
d) mixing of the shredded waste (1') inside the vessel (11), from a rotation of the worm (12) alternating in both directions;
e) in the case of introduction inside the hopper (6) of a new quantity of waste (1), any disinfection of the upper compartment (2) by projecting a disinfecting liquid, then introduction of waste (1) and driving of this waste (1) towards the intermediate compartment (3);
f) closing of the sliding valve (17) to isolate the hopper (6) and the intermediate (3) and lower (4) compartments, mixing of the shredded waste (1');
g) evacuation of the internal volume of the vessel (11) and projection of disinfecting liquid, for disinfecting of the shredded waste (1') and cutting tools (5), by circulation through a communication passage, arranged between the intermediate (3) and lower (4) compartments;
h) venting of the internal volume of the vessel (11), draining against the grid and conveying of the treated waste (1") towards the evacuation door (13), then emptying of exudates (32) through rotation of the worm (12) in the corresponding direction;
i) opening of the door (13) and evacuation of the treated waste (1") outside the vessel (11).

## Patentansprüche

1. Vorrichtung zur Parzellierung und zur Dekontaminierung oder zur Behandlung von Abfällen (1), vor allem von infektiösen medizinischen Abfällen, die auf einem Rahmen (24) montiert ist und eine mehrstufige Abfolge von Abteilen (2, 3, 4) umfasst, darunter ein oberes Abteil (2) zur Versorgung mit Abfällen, einen Trichter (6) umfassend, ein mittleres Abteil (3) zur Zerkleinerung, Schneidwerkzeuge (5) zur Parzellierung der Abfälle umfassend, und ein unteres Abteil (4) zur Desinfektion und Ableitung der Reststoffe,
wobei die Vorrichtung **dadurch gekennzeichnet ist, dass:**
- das obere Abteil (2) Mittel zur Beförderung (7) der Abfälle zum mittleren Zerkleinerungsabteil (3) umfasst, Mittel zur Umschließung des Innenvolumens des Trichters (6) zwischen der Einlassöffnung (14), den er umfasst, und seiner Auslassöffnung (15) zum mittleren Abteil (3), wobei jede dieser Öffnungen (14, 15) mit jeweiligen Verschlussmitteln (16, 17) ausgestattet ist, die zwischen einer entsprechenden Freigabestellung und einer Verschlussstellung der entsprechenden Öffnung bewegbar sind, und erste Mittel (19) zur Desinfektion der Abfälle (1) und des Trichters (6),
- das mittlere Abteil (3) durch einen Durchlass (8) direkt in das untere Abteil (3) mündet, wobei es mit diesem unteren Abteil (4) in ständiger Kommunikation ist, und zweite Desinfektionsmittel der Schneidwerkzeuge (5) umfasst,
- die Schneidwerkzeuge (5) in einem Gestell (23) umschlossen sind, das rahmenförmig mit Seitenwänden ausgebildet ist, in der Art eines Einschubs lösbar auf dem Rahmen (24) befestigt ist, das den Durchlass (8) begrenzt, und prinzipiell das mittlere Abteil (3) bilden, und
- das untere Abteil (4) einen Behälter (11) zur Aufnahme der zerkleinerten Abfälle aus dem mittleren Abteil (3) umfasst, wobei dieser Behälter (11) mit dritten Desinfektionsmitteln (21, 22) ausgestattet ist zur Behandlung der zerkleinerten Abfälle und zur Desinfektion der Schneidwerkzeuge (5) durch den Durchlass (8), und mit Mitteln (12) zur Ableitung der behandelten Abfälle zu einer Ableitungstür (13).

2. Vorrichtung nach Anspruch 1, **dadurch gekennzeichnet, dass** die Verschlussmittel der Einlassöffnung (14) des Trichters (6) einen an den Trichter (6) angelenkten Deckel (16) umfassen.

3. Vorrichtung nach Anspruch 1 oder Anspruch 2, **dadurch gekennzeichnet, dass** die Verschlussmittel der Auslassöffnung (15) des Trichters (6) einen Isolierschieber umfassen, der zwischen dem Trichter (6) und dem mittleren Abteil (3) angeordnet ist, einen mit dem Trichter (6) einerseits und dem mittleren Abteil (3) andererseits verbundenen Schieberkörper umfassend, wobei der besagte Schieberkörper eine durchgehende Öffnung zum Durchlass der Abfälle und der Schieber im weiteren einen Einschub (17) umfasst, der gleitend im Schieberkörper zwischen einer Verschlussstellung, in der die durchgehende Öffnung verschlossen ist und der Trichter (6) und das mittlere Abteil (3) voneinander isoliert sind, und einer Freigabeöffnung, in der die durchgehende Öffnung freigegeben ist und die Kommunikation zwischen dem Trichter (6) und dem mittleren Abteil (3) hergestellt ist, montiert ist.

4. Vorrichtung nach Anspruch 3, **dadurch gekennzeichnet, dass** der Schieberkörper um die durchgehende Öffnung herum mit einer aufblasbaren Umfangsdichtung (18) zur Abdichtung zwischen dem oberen Abteil (2) und dem mittleren Abteil (3) durch elastische Komprimierung des Einschubs (17) ausgestattet ist, der mit Hilfe der aufblasbaren Dichtung (18) gegen den Rahmen (24) gleitet, und um das Gleiten des Einschubs (17) durch seine Freigabe der Dichtung (18) ohne Druck zu erleichtern.

5. Vorrichtung nach einem der Ansprüche 2 bis 4, **dadurch gekennzeichnet, dass** die Mittel zur Beförderung der Abfälle aus dem oberen Abteil (2) in das mittlere Abteil (3) von einem Vortriebsorgan (7) in Form einer den Deckel (16) vollkommen durchquerenden Stange gebildet werden, das omnidirektional am Deckel (16) einerseits angelenkt ist und andererseits einen Spielraum der axialen Verschiebung besitzt, der ihm Zugang zum gesamten Innenvolumen des Trichters (6) und zu den Innenseiten desselben erlaubt, wobei das Organ mit dem Deckel (16) dicht verbunden ist.

6. Vorrichtung nach Anspruch 5, **dadurch gekennzeichnet, dass** das Vortriebsorgan (7) in einem zylindrischen Teil (40) gleitet, das mit einer elastischen Scheibe (41) verbunden ist, die selbst mit dem Deckel (16) verbunden ist, wobei dieses zylindrische Teil (40) eine interne Kammer (42) begrenzt, die vom Vortriebsorgan (7) durchquert wird und mit einem bakteriziden Produkt gefüllt ist.

7. Vorrichtung nach einem der Ansprüche 1 bis 6, **dadurch gekennzeichnet, dass** das obere Abteil (2) mit Mitteln zum Einlass eines Behandlungsfluids (Flüssigkeit, Gas oder Dampf) ausgestattet ist, von denen ein beliebiges Mittel mindestens erste Mittel (19) zum Einlass eines Desinfektionsfluids (20) zur Behandlung des Trichters (6) (Flüssigkeit, Gas oder Dampf) umfasst, und zweite Mittel zum Einlass von Desinfektionsflüssigkeit zur Behandlung der Abfälle (1) durch Baden.

8. Vorrichtung nach Anspruch 7, **dadurch gekennzeichnet, dass** die Verschlussmittel (16, 17) des Trichters (6) Rückhaltemittel der Desinfektionsflüssigkeit zur Behandlung der Abfälle (1) durch Eintauchen bilden.

9. Vorrichtung nach den Ansprüchen 2 und 7, **dadurch gekennzeichnet, dass** die ersten Einlassmittel eines Desinfektionsfluids (20) (Flüssigkeit, Gas oder Dampf) zur Behandlung des Trichters (6) von mindestens einer Düse (19) zur Projektion des entsprechenden Fluids (Flüssigkeit, Gas oder Dampf) gebildet werden, die durch den Deckel (16) in das Volumen des Trichters (6) mündet.

10. Vorrichtung nach einem der Ansprüche 2 bis 9, **dadurch gekennzeichnet, dass** der Deckel (16) als Kuppel ausgebildet ist, um das Fließen der Desinfektionsflüssigkeit (20) des Trichters (6) über seine Innenseite im Hinblick auf ihre Drainage in das Innenvolumen des Trichters (6) zu fördern.

11. Vorrichtung nach einem der Ansprüche 1 bis 9, **dadurch gekennzeichnet, dass** die Schneidwerkzeuge (5) in zwei parallelen Reihen von Schneidwerkzeugen (5) organisiert sind, wobei die Werkzeuge jeder Reihe auf einer tragenden Welle (27) zum rotierenden Antrieb, die der Werkzeugreihe zueigen ist, montiert sind, wobei die Werkzeuge auf ihren jeweiligen Wellen (27) im Verhältnis zu diesen Wellen in Rotation und Verschiebung starr montiert sind, wobei die Werkzeuge jeder Reihe gleichmäßig beabstandet voneinander angeordnet sind, so dass zwischen zwei aufeinanderfolgenden Werkzeugen ein Intervall ausgebildet ist, dessen Breite größer ist als die Dicke jedes Werkzeugs, und wobei die zwei Werkzeugreihen derart verschränkt angeordnet sind, dass die Werkzeuge jeder Reihe in die Intervalle zwischen den Werkzeugen der anderen Reihe eindringen.

12. Vorrichtung nach Anspruch 11, **dadurch gekennzeichnet, dass** die zwei Antriebswellen (27) von außerhalb des Rahmens (24) im gegenläufiger Richtung zueinander und mit unterschiedlichen Drehgeschwindigkeiten rotierend angetrieben werden.

13. Vorrichtung nach Anspruch 11 oder nach Anspruch 12, **dadurch gekennzeichnet, dass** die Schneidwerkzeuge (5) jeder Reihe durch Abstandshalter (26) voneinander getrennt sind, die auf der entsprechenden Welle angebracht sind.

14. Vorrichtung nach einem der Ansprüche 11 bis 13, **dadurch gekennzeichnet, dass** die zweiten Desinfektionsmittel mindestens einen Kanal (28) zur Beförderung eines Desinfektionsfluids (Flüssigkeit, Gas oder Dampf) umfassen, der durch mindestens eine der Seitenwände des Gestells (23) ausgebildet ist in radialer Richtung zu den Schneidwerkzeugen (5) und sich verlängert, um in die Intervalle zwischen den Werkzeugen zu münden, so dass die Desinfektionsflüssigkeit direkt von außerhalb des Gestells (23) heranführbar ist und gegen die Seitenwände der Schneidwerkzeuge (5) in einer Projektionsrichtung in einer parallelen Ebene zu diesen Seiten projizierbar ist.

15. Vorrichtung nach einem der Ansprüche 1 bis 14, **dadurch gekennzeichnet, dass** die dritten Desinfektionsmittel, die den Behälter (11) des unteren Abteils (4) ausstatten, Mittel zur Erzeugung eines Vakuums im Innenvolumen des Behälters (11) umfassen.

16. Vorrichtung nach einem der Ansprüche 1 bis 15, **dadurch gekennzeichnet, dass** die dritten Desinfektionsmittel (22), die den Behälter (11) des unteren Abteils (4) ausstatten, Mittel zum Einlass eines Desinfektionsfluids (21) unter Druck in diesen Behälter (11) umfassen.

17. Vorrichtung nach einem der Ansprüche 1 bis 16, **dadurch gekennzeichnet, dass** die dritten Desinfektionsmittel, die den Behälter (11) des unteren Abteils (4) ausstatten, in Rührmitteln (12) der zerkleinerten Abfälle (1') zwecks ihres Mischen während der Desinfektionsphase inbegriffen sind.

18. Vorrichtung nach Anspruch 17, **dadurch gekennzeichnet, dass** die Rührmittel von einer Schnecke (12) gebildet werden, die alternativ in zwei Drehrichtungen bewegbar ist, um die Anpassung der aktiven Mischzeit an die Zuführung von zerkleinerten Abfällen (1') aus dem mittleren Abteil (3) zu erlauben.

19. Vorrichtung nach Anspruch 18, **dadurch gekennzeichnet, dass** die Mittel zur Ableitung der zerkleinerten Abfälle (1') von der Schnecke (12) gebildet werden zwecks ihrer Beförderung zur Ableitungstür (13) mittels einer kontinuierlichen Rotation der Schnecke (12) in die entsprechende Richtung.

20. Vorrichtung nach einem der Ansprüche 1 bis 19, **dadurch gekennzeichnet, dass** sie ein Gitter (33) zum Schleudern der behandelten Abfälle (1") umfasst, das am Ausgang des Behälters (11) angeordnet ist, und Mittel (31) zur Ableitung der Exsudate (32), die an der Basis des Behälters (11) angeordnet sind.

21. Methode zur Behandlung von Abfällen (1) durch Parzellierung und Desinfektion, die eine Vorrichtung nach Anspruch 1 umsetzt, **dadurch gekennzeichnet, dass** sie für einen gegebenen Zyklus der Behandlung der Abfälle (1) darin besteht, im wesentlichen die folgenden Schritte durchzuführen:
- Öffnen des oberen Abteils (2) zur äußeren Umgebung zwecks Einleitung der Abfälle (1), danach Verschließen der Einlassöffnung (14) des Trichters (6) und Beförderung der Abfälle (1) zum mittleren Abteil (3),
- Zerkleinern und kontinuierliche Einleitung der zerkleinerten Abfälle (1') in den Behälter (11) bis zu einer gewünschten Menge, die der Menge der Abfälle (1) entspricht, die zuvor in den Trichter (6) eingeführt wurde,
- Isolierung des oberen Abteils (2) durch Verschließen der Auslassöffnung (15) des Trichters (6),
- gleichzeitige Operationen zur Desinfektion der Werkzeuge und der zerkleinerten Abfälle (1'), die sich im Behälter (11) befinden.

22. Methode nach Anspruch 21, die eine Vorrichtung nach den Ansprüchen 1 bis 19 umsetzt, **dadurch gekennzeichnet, dass** sie insbesondere darin besteht, die Schritte zur Durchführung eines Zyklus' zur Behandlung von Abfällen (1) durchzuführen, der die folgenden aufeinanderfolgenden Schritte umfasst:
a) Desinfektion des oberen Abteils (2) durch Projektion von Desinfektionsflüssigkeit vor dem Öffnen des Verschlussdeckels (16) der Einlassöffnung (14) der Abfälle (1) des Trichters (6),
b) Öffnen des Deckels (16) und Einleiten der Abfälle (1) in den Trichter (6), danach Schließen des Deckels (16),
c) Beförderung der Abfälle (1) mit dem Vortriebsorgan ab dem Trichter (6) zu den Schneidwerkzeugen zu deren Zerkleinerung und Einleiten der zerkleinerten Abfälle (1') in den Behälter (11),
d) Mischen der zerkleinerten Abfälle (1') im Behälter (11) durch eine Rotation der Schnecke (12) abwechselnd in die zwei Richtungen,
e) bei erneutem Einleiten einer neuen Menge von Abfällen (1) in den Trichter (6) Desinfektion des oberen Abteils (2) durch Projektion einer Desinfektionsflüssigkeit, danach Einleiten von Abfällen (1) und Vortrieb dieser Abfälle (1) zum mittleren Behälter (3),
f) Schließen des gleitenden Einschubs (17), um den Trichter (6) vom mittleren Abteil (3) und vom unteren zu isolieren, danach Mischen der zerkleinerten Abfällt (1'),
g) Erzeugen eines Vakuums im Innenvolumen des Behälters (11) und Projektion einer Desinfektionsflüssigkeit zur Desinfektion der zerkleinerten Abfälle (1') und der Schneidwerkzeuge (5) durch eine Zirkulation der Desinfektionsflüssigkeit durch den Kommunikationsdurchlass, der zwischen dem mittleren und unteren Abteil eingerichtet ist,
h) Versetzen des Innenvolumens des Behälters (11) unter atmosphärischen Druck, Schleudern der Abfälle (1) gegen das Gitter und Beförderung der behandelten Abfälle (1") zur Ableitungstür (13), danach Ablassen der Exsudate (32) durch Rotation der Schnecke (12) in die entsprechende Richtung,
i) Öffnen der Ableitungstür (13) und Ableitung der behandelten Abfälle (1") außerhalb des Behälters (11).
